# EUROPEAN PATENT APPLICATION

(11) **EP 2 072 117 A1**
(43) Date of publication of application: **24.06.2009**
(21) Application number: 07150151.4
(22) Date of filing: 19.12.2007
(51) Int. Cl.: B01J 20/28, A61M 1/16, C02F 1/28

(54) **Sorbent material**

(71) Applicant: Nederlandse Organisatie voor Toegepast- Natuurwetenschappelijk Onderzoek TNO, 2628 VK Delft (NL)
(72) Inventor: Schilthuizen, Stephanus Franciscus, 5056 MG Berkel-Enschot (NL); Batenburg, Lawrence Fabian, 5652 EJ Eindhoven (NL); Simonis, Frank, 5688 RX Oirschot (NL); Vercauteren, Franky Flory, 5644 DK Eindhoven (NL)
(74) Representative: Hatzmann, Martin

(57) **Abstract**

The present invention relates to a sorbent material for removing substances from an aqueous liquid comprising a nanostructured sorption material captured in a porous polymer matrix, wherein the pores in said matrix are of a size that allows the entry into said matrix of substances sought to be removed from said liquid while preventing the escape of said nanostructured sorption material or preventing the entry into said matrix of substances not sought to be removed from said liquid.

## Description

### FIELD OF THE INVENTION

The present invention is in the field of liquid purification, more in particular to the selective removal of toxins from biofluids, blood, blood plasma, and from dialysates for hemodialysis and peritoneal dialysis. The invention relates to a sorbent material enabling a high degree of removal of undesired substances from liquids such as toxins from biofluids and from the blood of a patient, and to methods of removing undesired substances from liquids such as toxic substances from biofluids and blood using the sorbent material. The invention further relates to functional additives for dialysate fluids and to filter pads comprising the sorbent material for use in dialysis systems and artificial kidneys.

### BACKGROUND OF THE INVENTION

Hemodialysis (HD) and peritoneal dialysis (PD) are methods of removing toxic substances (impurities or wastes) from the blood when the kidneys are unable to do so sufficiently. Dialysis is most frequently used for patients who have kidney failure, but may also be used to quickly remove drugs or poisons from the body in acute situations. This technique can be life saving in people with acute or chronic kidney failure. Best known is hemodialysis, which works by circulating the blood along special filters outside the body in a dialysis machine. Dialysis works on the principles of the diffusion and osmosis of solutes and fluid across a semipermeable membrane (which is part of the dialyser or filter module). Blood flows by one side of a semipermeable membrane, and a dialysis fluid or dialysate flows in a countercurrent direction to the blood flow at the opposite side of the membrane. Smaller solutes (below a certain molecular cut-off) and water can pass through the membrane and since the concentration of undesired (toxic) small solutes (for example potassium, calcium, and urea) is high in the blood, but low or absent in the dialysate, they will diffuse from the blood across the dialyser into the dialysate. Constant replacement of the dialysate ensures that the concentration of these undesired solutes remains low therein. The dialysis fluid thus removes the toxins from the blood and is generally discarded as waste dialysate. The chemical imbalances and impurities of the blood are being brought back in minimal balance and the blood is then returned to the body. The efficacy of hemodialysis is approximately 10-15%, which means that 10-15% of the toxins are being removed from the blood. Typically, most patients undergo hemodialysis for three sessions every week, usually in a dialysis center. Each session lasts normally 3-4 hours. This is very inconvenient, and the physical and social side effects of dialysis to the patients are a great concern. However, studies have shown that both increased treatment length and frequency would be clinically beneficial. This is only possible when people are dialyzing at home.

In peritoneal dialysis, the dialysis fluid containing minerals and glucose or dextrose is run through a soft catheter into the peritoneal cavity, the abdominal body cavity around the intestine, where the peritoneal membrane acts as a semipermeable membrane. The dialysate is left in the peritoneal cavity for a sufficient period of time to absorb waste products, and is then drained from the body and discarded. This exchange cycle is normally repeated 4-5 times during the day, (sometimes more often overnight with an automated system) and takes about 30 to 40 minutes. Osmosis is again the driving force behind the process; the dialysate contains a high concentration of glucose or dextrose, and the resulting osmotic pressure causes fluid to move from the blood into the dialysate. As a result, more fluid is drained than was instilled. Peritoneal dialysis is less efficient than hemodialysis, but because it is carried out for a longer period of time the net effect in terms of removal of waste products and of salt and water are similar to hemodialysis. Peritoneal dialysis is usually carried out at home by the patient. It frees patients from the routine of having to go to a dialysis clinic on a fixed schedule multiple times per week, and it can be done while travelling with a minimum of specialized equipment.

In order to provide portable dialysis devices that will allow patients to engage in normal daily activities, artificial kidneys have been developed and such systems are the subject of co-pending applications. The present invention relates to the problem of the low efficacy of dialysis systems and methods.

As noted above, the current efficacy of both the hemodialysis and peritoneal dialysis treatments is very low, the procedures are time consuming and especially in case of hemodialysis, require high volumes of purified water (30-50 liters per treatment) and dialysis components. The poor performance is due to the slow diffusion process of toxins through the hemo- or peritoneal membrane, the limited osmotic pressure difference over the membrane and the increase in toxin concentrations in the dialysate fluid which further slows diffusion. In order to increase the efficiency of dialysis, it is desirable to maintain the trans-membrane concentration gradients of waste metabolites as high as possible.

In one form, the efficacy of the dialysis treatment can be improved by continuous regeneration of the dialysate by means of an absorbent, usually activated carbon. In a dialysis kidney machine, the dialysis fluid is regenerated when it is to be recycled into the dialyser. Dialysis kidney machines with dialysate regeneration encountered in the prior art include for instance those described in GB 1 406 133, and US 2003/0097086. GB 1 406 133 discloses an artificial kidney of the recycle type having an improved adsorbent comprising activated carbon and alumina. US 2003/0097086 discloses a portable dialysis device comprising dialyzers connected in series that utilize dialysate, and further comprising a plurality of contoured sorbent devices, which are connected in series and are for regenerating the spent dialysate. As adsorption materials for regeneration of the spent dialysate, activated charcoal, urease, zirconium phosphate, hydrous zirconium oxide and/or activated carbon are provided.

In other forms, systems that absorb toxic substances directly from the blood have also been proposed. US 2004/0147900 discloses a cartridge for treating medical or biological liquid, in particular blood, consisting of a compartmentalized container, wherein each compartment contains adsorbing particles. The adsorption materials proposed are essentially those disclosed in US 2003/0097086 described above, and thus may effectively remove urea from blood.

As noted above, the adsorbent for regenerating the dialysate is usually activated carbon. However other adsorbents have been proposed for the removal of substances from dialysis fluids or ultrafiltrate. US 3,874,907, for instance, discloses microcapsules consisting essentially of a crosslinked polymer containing sulphonic acid groups and coated with a polymer containing quaternary ammonium groups, for use in an artificial kidney. Examples of the sulphonated polymer include sulphonated styrene/divinyl benzene copolymer and examples of the coating polymer include those obtained by polymerization of for instance vinyldimethylamine monomers. Shimizu *et al*. (Nippon Kagaku Kaishi (1985), (6), 1278-84) described a chemisorbent composition for the removal of urea from dialysis fluid or hemofiltrate for use in an artificial kidney. The chemisorbent is based on dialdehyde starch (DAS)-urease conjugates and 4,4'-diamidinodiphenylmethane (DADPM).

US 4,041,233 discloses an absorbent material for removing urea molecules from dialysate fluids, which material is insoluble in dialysate utilized in an membrane-based artificial kidney system. The material is based on a modified polysaccharide such as cellulose or starch, containing dialdehydes derived by periodate oxidation of the amylose C₂-C₃ hydroxyl groups. This absorbent material has an absorption capacity of at least 1% urea based on weight of absorbent which is still lower than a urease-zirconium phosphate system with an urea absorption of about 2%.

US 4,013,564 discloses a device for removing undesirable materials from blood. The device consists of a hemofilter membrane having a pore size of 0.05 to 20 µm which envelops a particulate adsorbent or treating/treatment agent. The blood plasma, separated from the blood cells, flows through the hemofilter and passes the treating agent and is subsequently re-combined with the cellular fraction of the blood. The treating agent may comprise activated carbon, adsorbent resins, alumina, zirconium oxide, zirconium phosphate, oxidized starch (cellulose oxide), ion exchange resins, immobilized enzymes such as urease or asparaginase, liver tissue, urine-dependent bacteria, nutrients and hormones. The purpose of the membrane is i.a. to prevent the particulate treating agent from entering the blood, while allowing separation of blood cell from plasma. According to US 4,013,564 the particles of the particulate treating agent should not have smaller dimensions than the lower limit of the membrane pore size (0.05 µm).

US 6,579,460 describes a composite for removing toxic substances from blood or dialysate comprising a mixture of an anion exchange resin and a microporous cation exchange resin of zirconium metallate or titanium metallate. The anion exchange resin may be a basic clay or a layered double hydroxide. The composite may be prepared in the form of sorbent particles in the range of 20 to 600 micron diameter.

GB 1 552 248 describes the removal of urea from dialysing liquid using granular oxidized starch having a particle size of 0.5-1 cm.

WO 03/051422 discloses a system for peritoneal dialysis comprising sorbent devices having replaceable cartridges containing the generally known sorbent materials such as activated charcoal, urease zirconium phosphate, and hydrous zirconium oxide.

WO 02/43859 discloses sorbent cartridges for cleaning dialysis fluids comprising granular activated carbon, sodium zirconium carbonate particles of 30-50 microns, and alumina particles of 20-40 microns.

US 5,284,470 describe an artificial kidney with a blood/plasma separator having holes of 0.4-3.0 microns and an urease coating.

In conclusion, the prior art discloses absorber units, absorbent cartridges for regeneration of dialysis fluids and discloses sorbent materials for use in such units and cartridges, wherein various substances are used as the sorbent material.

The problem with the sorbent systems of the prior art is however, that due to limited sorption capacity of the materials used in those systems, vast quantities of sorbent material are needed to provide for efficient removal of toxic substances. This still results in relatively large absorber units through which the dialysate fluids need to be pumped, and consequentially relatively large pumping equipment.

It is an object of the present invention to overcome the problems associated with the large absorber devices of the prior art and to provide a direct and convenient toxin removing system without the need of dialysate regenerating machines.

### SUMMARY OF THE INVENTION

The inventors have surprisingly discovered a sorbent material that is extremely suitable for removing substances from biofluids, blood and bloodplasma and from hemodialysis and peritoneal fluid, which sorbent material can for instance be used in a wearable artificial kidney or which sorbent material can be used as a direct additive to a dialysate (a so-called sorption-additive) or any other fluid, from which the removal of specific substances, in particular toxic substances, is required. In addition to purifying blood, blood plasma and dialysis fluid, the materials in aspects of the present invention can also be used for purification of other biofluids such as fluids extracted from the body that are subject for detailed analysis such as DNA profiling including PCR magnification. The removal of unwanted components such as inonic solutes and proteins will simplify and fasten the preparation and analysis procedure.

In a first aspect, the present invention relates to a sorbent material for removing substances from an aqueous liquid comprising a nanostructured sorption material captured in a porous polymer matrix, wherein the pores in said matrix are of a size that allows the entry into said matrix of substances sought to be removed from said liquid while preventing the escape of said nanostructured sorption material or preventing the entry into said matrix of substances not sought to be removed from said liquid.

The sorbent material has a high sorption capacity for the removal of toxic substances from hemodialysis and peritoneal fluid, for absorbing ionic solutes and small-sized or middle-sized molecules. The sorbent material allows for the use of small blood and bloodplasma volumes allowing direct filtration of blood or bloodpalsma and also reduces the dialysate volume being capable of maintaining a high osmotic pressure difference (high concentration gradient of waste metabolites) between dialysate and sorbent material. This allows for higher clearance rates, typically 100% higher than conventional systems without the additive.

Said sorbent material comprises an absorbing, adsorption, ion-exchange and/or surface crystallisation material in the form of nanostructured material, such as clay minerals, in particular nanoclays, preferably a smectite group nanoclay ,including but not limited to pyrophyllite, talc, vermiculite, sauconite, saponite, nontronite, montmorillonite, hectorite, beidellite, magadiite, kenyaite, volkonskonite, sobockite, stevensite, svinfordite, synthetic flouromica and combinations of the foregoing), layered double hydroxide (anionic clays or hydrotalcites), nanoporous silica's, nanoporous or layered alumina silicates (such as zeolites), nanosized and /or activated graphite, cyclo-dextrines, crystallisation seeds or combinations thereof.

The nano sized particles are dispersed in a polymer matrix. The polymer matrix is preferably a crosslinked modified biopolymer biopolymer.

In a preferred embodiment, the nanostructured sorption material in a sorbent material of the invention is selected from the group consisting of nanoparticles or nanocrystalline materials, nanoporous materials, nanocomposites, nanoclays and nanofibers, and any combination thereof.

In the case that a nanocrystalline material is used, said nanocrystalline material is preferably a nano hydrotalcite. Very suitable nanohydrotalcites are nanocrystalline materials based on Fe(OH)ₓ, Mg₂Fe(OH)₆.OH, MgCl₂.6H₂O, or Mg.OH.

In the case that a nanoclay is used, said nanoclay is preferably as exfoliated nanoclay. Most preferably (Na₀,₂,Ca₀,₁)(Al,Mg)₂(Si₄O₁₀)(OH)₂.

In the case that a nanoporous material is used, said nanoporous material is preferably selected from zeolites, mesoporous systems, and metal organic frameworks.

The porous polymer matrix in a sorbent material according to the invention is preferably based on a cross-linked polymer and/or a charged polymer. The polymer is preferably a biopolymer, suitably selected from carbohydrates and proteins. A particularly preferred polymer is oxidized crosslinked starch, in particular as exemplified in the examples.

A sorbent material according to the present invention is preferably adapted to be suitable for the removal of substances from aqueous liquids. To this end, materials used in the sorbent material and which are not intended specifically to be released therefrom, are provided in a form wherein they are not soluble in said liquid, or in a from wherein the integrity or performance of the material is not deteriorated due to exposure to aqueous liquids. The aqueous liquid is preferably dialysate fluid, blood or bloodplasma.

A sorbent material according to the present invention is preferably adapted or suited for the removal of toxic substances from said liquid. Toxic substances are preferably selected from potassium, phosphate, urea, creatinine, beta2-microglobulin (β2M), and albumin bound toxins.

A sorbent material according to the present invention may further comprise means for supplementing said dialysate fluid and/or said (purified) blood plasma with at least one substance selected from the group consisting of vitamins, minerals, anticoagulants, anti microbial agents and medicaments. These substances (in particular vitamins C and B12, heparin and erythropoietin) may suitably function as surfactants on the nanostructured sorption material and the polymer matrix. The term means should be interpreted broadly in the sense that said vitamins, minerals, anticoagulants, anti microbial agents and medicaments are provided to the sorbent material in such a way that they can be released from said sorbent material upon contact with the aqueous liquid.

A sorbent material according to the present invention may be used in various applications. In particular applications are foreseen for removing toxins, small and middle-sized molecules from a dialysate or a patient's blood or blood plasma; for use as an additive to dialysate fluids; and for use in a hemodialysis system or peritoneal dialysis system. An additive of the present invention comprises a sorbent material as defined herein as the active substance-sorbing ingredient.

In another aspect, the invention provides a filter pad comprising a sorbent material according to the present invention. The filter pad may optionally further comprise a hemofilter. Very suitably, the sorbent material is enveloped by the hemofilter material. In a preferred embodiment of a filter of the invention, the sorbent material is provided in the form of dried granules having a mean size over the range 250 microns to 1500 microns (in dried form). These granules will swell upon contact with the aqueous liquid, and the gel is suitably retained in the filter pad by the hemofilter membrane.

In another aspect, the invention provides a method for removing substances from an aqueous liquid comprising the step of exposing said liquid to a sorbent material according to the present invention or filtering said liquid through a filter pad according to the present invention.

### BRIEF DESCRIPTION OF THE DRAWINGS

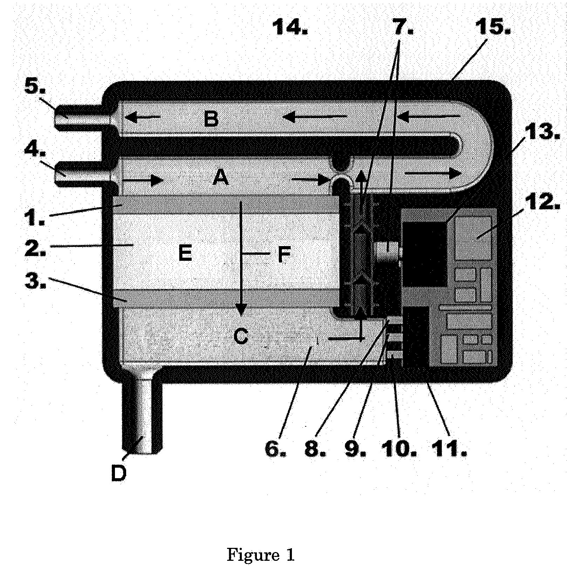

Figure 1 shows a cross-sectional presentation of a device wherein the sorbent material according to the invention may suitably be used. The device as depicted in Fig. 1 comprises a sorption filter (2), fitted between two micron sized (hemo) filters (1 and 3). These parts (1), (2) and (3) may be provided to form a pad as separate parts or combined into a single part, wherein the micron sized (hemo) filters envelope the sorption filter. Essentially, the sorbent material of the present invention may be (part of) the sorption filter. The skilled person will understand that the filter pad may take various embodiments as described herein above. The device as depicted in Fig. 1 further comprises a blood inlet (4), which may be connected to vascular access and tubing; a blood outlet (5), which is provided for recovery of blood cells and filtered blood plasma; a blood plasma outlet (6) for the recovery of filtered blood plasma flowing out of the sorption filter and bottom hemofilter; a (micro)-pump (7); at least one sensor (8), (9), and/or (10) for measuring salt and/or small and/or middle-sized molecule content, etc.; electronic components (11) (e.g. for digital signal processing (DSP), interfaces for sensors); a RF-wireless radio module (12) providing a link to a medical consultant or computer; a power supply for the device (e.g. incl. power supply for the pump) and accompanying electronics and mechatronic part for the pump (13); a housing, optionally comprised of a monolithic structure, but preferably comprising a first part (14, not drawn) and a second (15) part, which first and second part are separably connected to each other possibly combined with integrated or separate liquid and airtight sealing gaskets to provide the enclosure for the various components of the device, and which, when separated, provide access to the interior of the device. The letters A-F indicate: blood patient (A); blood return (B); cleansed blood plasma (C); excess water outlet (D); sorption filter pad (E); and blood plasma flow across sorption filter pad (F).

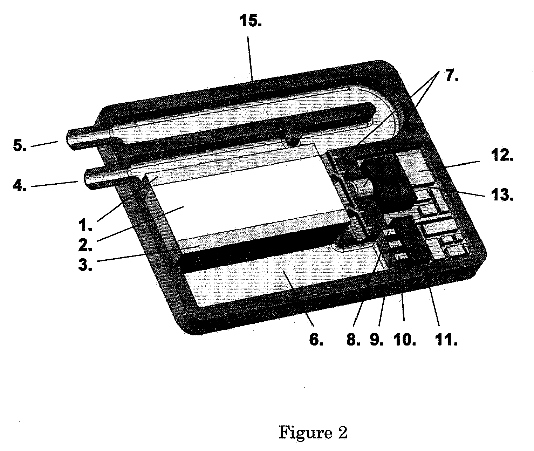

Figure 2 shows an opened out 3D-model of a device described in Figure 1.

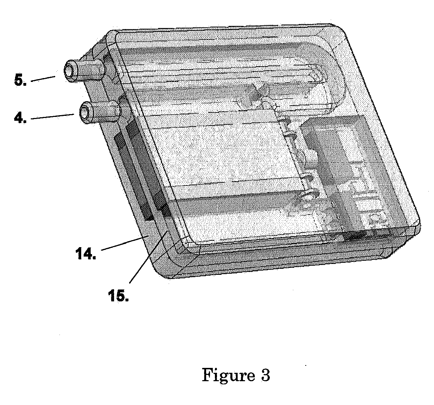

Figure 3 is an artist's impression of an assembled device as described in Figures 1 and 2.

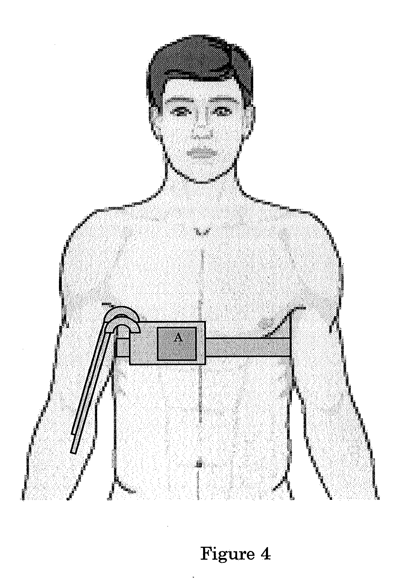

Figure 4 shows an example of a wearable artificial kidney system comprising the filter pad of the invention (indicated by the letter A) fitted to a belt and provided with conduits for blood feed and blood return.

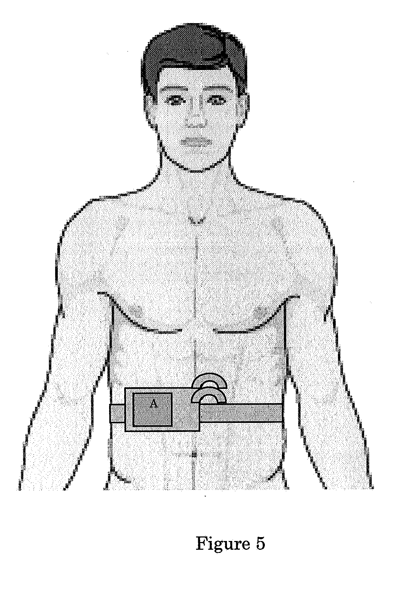

Figure 5 shows an example of a wearable peritoneal dialysis system comprising the filter pad of the invention (indicated by the letter A) fitted to a belt and provided with conduits for feed and return of peritoneal dialysis fluid.

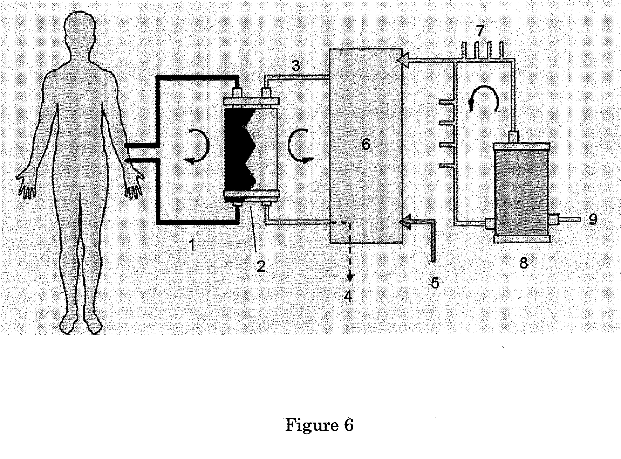

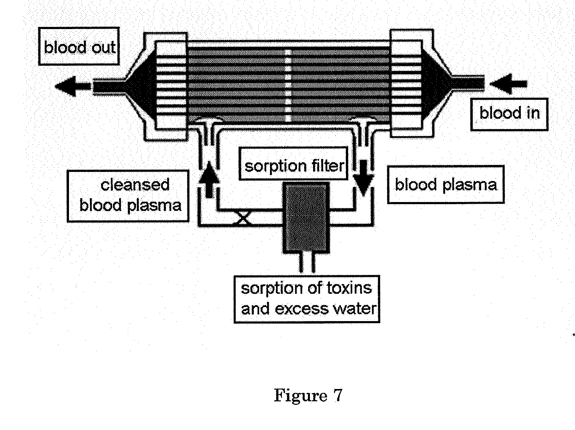

Figure 6 shows the prior art hemodialysis procedure indicating the extracorporeal circuit (1); the dialyzer (2); the dialysis fluid circuit (3); waste outlet (4); concentrated dialysate input (5); dialysis machine (6); treated water (7); water purification (8); and source water (9). The system uses in general 120 liters of dialysate per treatment, employs a low osmotic pressure difference, uses a low to medium flux hemofilter and removes only small molecules. All these items may be replaced by a hemofiltration unit as shown in Figure 7 below.
Figure 7 shows a hemofiltration unit of the present invention comprising a blood inlet and outlet, a hemofilter for separating blood cells from plasma, and a blood plasma circuit running across a sorption filter according to the present invention for the sorption of toxins and excess water. Such a system features direct filtration of blood plasma and extraction of compounds from the plasma, consumes no dialysate and requires no water treatment equipment and uses high flux hemofilter through which albumin can pass. The a sorption pad filter system removes not only the "standard" toxins from the blood such as small molecules like potassium, phosphate, and urea, but also toxic substances which are not removed by prior art hemodialysis systems. In particular, the sorption pad of the invention also clears middle molecules such as creatinine, beta2-microglobulin (β2M), protein- (albumin) bound toxins such as bilirubin. This results in much better health condition of ESRD patients.

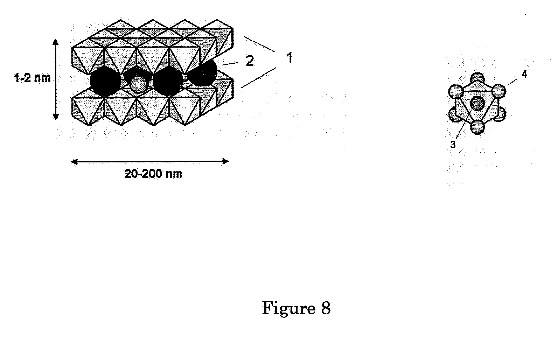

Figure 8 shows the layered crystal structure of hydrotalcite-like compounds (layered double hydroxides; LDHs): numbers indicate hydroxide layers ([M^{II}₁₋ₓM^{III}ₓ(OH)₂]^{x+}) (1) and interlayer containing An- anions (large spheres) and water molecules (small spheres) (2); individual hexahedral structure of the metal hydroxide is shown on the right indicating the metal (II or III) atom (3) and hydroxide groups (4).

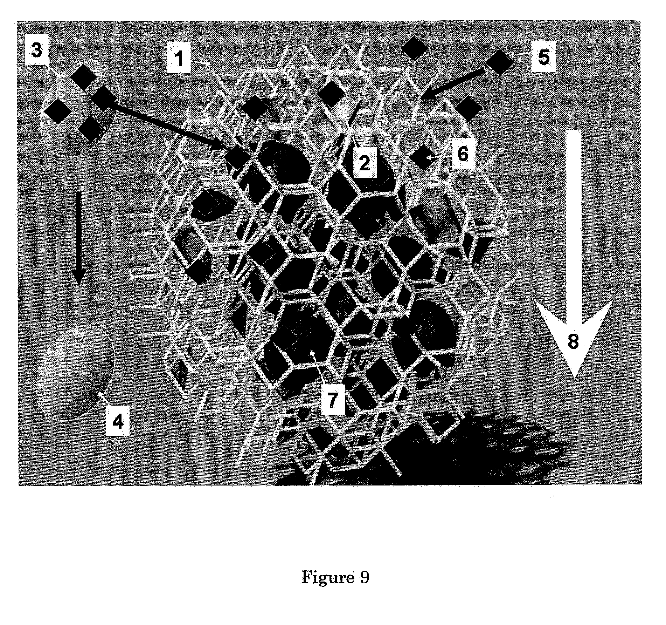

Figure 9 shows a sorption bead or sorption granule according to the present invention comprising a nanoporous (biopolymer) matrix functionalised with nanoparticles (nanoclays or hydrotalcites, in particular precipitated metal hydroxides) creating a high, selective sorption power. Numbers indicate the following (1): nanoporous biopolymer, in particular a modified starch with tuneable porosity for selective uptake of small and middle molecules; (2): sorption nanoclay or hydrotalcite, preferably functionalized for sorption of anions, cations and (toxic) proteins; (3): albumin with bound toxins; (4): cleared albumin; (5): free toxins; (6) and (7): trapped toxins, sorbed molecules, ionic solutes and small molecules at the inside, middle molecules at the outside of the bead; (8): blood flow.

### DETAILED DESCRIPTION OF THE INVENTION

The term "sorption" as used herein, refers to both adsorption and absorption. Adsorption is a process that occurs when a gas or liquid or solute (called adsorbate) accumulates on the surface of a solid or more rarely a liquid (adsorbent), forming a molecular or atomic film (adsorbate). It is different from absorption, where a substance diffuses into a liquid or solid to form a "solution". The term sorption encompasses both processes, while desorption is the reverse process.

The term "small-sized molecules", as used herein, refers to molecules with a molecular weight lower than 500 Da, such as uric acid, urea, guanidine, ADMA, creatinine.

The term "middle-sized molecules", as used herein, refers to molecules with a molecular weight between 500 Da and 5000 Da, such as end products from peptides and lipids, amines, amino acids, protein bound compounds, cytokines, leptins, microglobulins and some hormones.

The term "nanoporous materials" refers to materials having pores that are by definition roughly in the nanometre range, that is between 1x10⁻⁷ and 0.2x10⁻⁹ m and includes reference to the 3 categories set out by IUPAC of microporous materials (such as zeolites) having pore sizes of 0.2-2nm; mesoporous materials having pore sizes of 2-50nm; and macroporous materials having pore sizes of 50-1000nm.

The term "ionic solutes", as used herein, refers to components such as phosphates, sulphates, carbon hydrates, chlorides, ammonia, potassium, calcium, sodium.

### The sorbent material

The present invention relates to a sorbent material, comprising a nanostructured sorption material captured in a porous polymer matrix. The sorbent material is preferably provided in the form of (dried) particles. The term "captured" refers to the fact that the nanostructured sorption material cannot "leak" out of the polymer matrix but is immobilized therein. This can be accomplished by covalent bonding of the nanostructured material to the polymer matrix, by electrostatic interactions between the nanostructured material and the polymer matrix, by intercalation of the polymer matrix into the nanostructured material, by dispersion of the (exfoliated) nanostructured material in the solid phase of the porous matrix, but preferably by trapping of the nanostructured material in the porous polymer matrix, wherein the term trapping refers to the fact that the movement of the nanostructured material through the pores of the porous polymer matrix is size restricted.

### The nanostructured sorption material

The nanostructured material exhibits sorption capacity of various substances, based on ion-exchange, surface nucleation (surface crystallisation) activity and/or surface adsorption activity.

The sorption material is preferably functionalized, such as to exhibit improved sorbing properties of toxic substances such as urea as compared to the non-functionalized material. The sorption material in the present invention is a nanomaterial, meaning that the material consists of particles or contains particles with a size of preferably 100 nanometres or less in order create a large specific surface area. The nanomaterials will enable very high sorption efficiency and therefore enable a small sized, lightweight and ultimately wearable dialysis machine.

Typically the nanostructured sorption material will exhibits ion exchange or intergallery or intergalleries filtering. Intergalleries are the spaces between the single crystals of minerals which make up the nanostructured material. Preferably, ion exchange relates to anion exchange and/or cation exchange, with anions such as Cl⁻, Br⁻, I⁻, SO₄²⁻, PO₃³⁻, NO₃⁻, CO₃²⁻, RCOO⁻ (carboxyl group, wherein R is a organic substituent) etc., and cations such as K⁺, Ca²⁺, Na⁺, Cu²⁺, Zn²⁺, Al³⁺, NH₃⁺, RₓNH_{y}⁺ (an aliphatic amine, wherein R is a organic substituent, x is en integer from 1 to 3, and y is an integer from 0 to 2), etc.
In general, suitable nanostructured sorption materials with ion-exchange and/or surface nucleation activity are layered double hydroxides (anionic clays). Layered double hydroxides (LDH's) are a class of ionic lamellar solids with positively charged layers with two kinds of metallic cations and exchangeable hydrated gallery anions. Layered double hydroxides (LDHs) comprise an unusual class of layered materials with positively charged layers and charge balancing anions located in the interlayer region. Phyllosilicates usually have negatively charged layers and cations in the interlayer spaces. LDHs are therefore also referred to as anionic clays or as hydrotalcite-like compounds. Hydrotalcite-like compounds (HT) can be represented by the following formula: [Mg₁₋ₓ Alₓ (OH)₂]^{x+} [A_{x/n}ⁿ⁻. mH₂0]^{x-}, wherein 0 ≤ x < 0.33, and Aⁿ⁻ is an exchangeable anion having a valence of n. These compounds are similar to the mineral hydrotalcite, Mg₆Al₂(OH)₁₆CO₃.4H₂0. LDH's may have different cations, such as Mg, Mn, Fe, Co, Ni, Cu and/or Zn as divalent cations, and Al, Mn, Fe, Co, Ni, Cr and/or Ga as trivalent cations. Processes for the production of synthetic LDHs are *inter alia* described in US 3,539,306 and US 3,650,704.

A layered double hydroxide material (LDH) for use in aspects of the present invention is composed of small crystalline sheets of sub-micron dimensions, between which anions are located. US 4,904,457, which is incorporated herein by express reference thereto, describes possible methods of preparation for a synthetic layered double hydroxide. Preferably, the layered double hydroxide has a large contact surface and an ion exchange capacity of 20 to 600 milliequivalents per 100 grams. An LDH preferably used is a hydrotalcite or a hydrotalcite-like material, because these materials can be readily prepared synthetically and the desired properties can be closely controlled during synthesis as will be readily determined by those of ordinary skill in the art.

In general, particularly suitable nanostructured sorption material with ion-exchange and/or surface nucleation activity is a hydrotalcite comprised of a mixture of magnesium and aluminium hydroxide in the form of a layered, anion intercalation compound. It is generally utilized as acid adsorbent, catalyst carrier, and anion exchanger. Particularly preferred hydrotalcites are Mg₂Fe(OH)₆.OH and Mg.OH. It should be noted that hydrotalcites used in aspects of the present invention need not be layered, but may be provided in the form of nanocrystalline materials.

In general, suitable nanostructured sorption materials are small nanosized particles, that induce surface crystallisation by mimicking the morphology of the chemical species to be removed such as urea crystals or urea coated particles. These heterogeneous seeds are generally used in filter assisted crystallisation processes. The sorbing process is based on induction of crystallisation of the species to be removed and subsequent filtering of the crystallites.

"Nano sized" as used herein, refers to a size of approximately 1-1000 nm, more preferably 1-100 nm.

A suitable sorption material with ion-exchange and/or surface nucleating activity is a clay, in particular a nanoclay. The clay material may be of a natural or synthetic nature. Preferably, the clay has a large contact surface. Very suitable are clay types based on layered silicates, such as layered phyllosilicate composed of magnesium and/or aluminum silicate layers which are each about 7-12 Å in thickness. Especially preferred are smectite-like clay minerals, such as montmorillonite, saponite, hectorite, fluorohectorite, beidellite, nontronite, vermiculite, halloysite and stevensite. Preferred are smectite, hectorite, saponite, and montmorillonite.

The term "nanoclay" refers to a clay (hydrous aluminium phyllosilicate), preferably from the smectite family, having a unique morphology, featuring one or more dimensions, such as length, width, or thickness, in the nanometer size (10⁻⁹ meter). The nanoclay may be described as consisting of extremely fine platelets, each having a high aspect ratio and large surface area. Montmorillonite clay is the most preferred smectite clay. The smectite clay particle, or platelet, is a sheet-like structure separated by interlayers. The interlayers may be intercalated with an intercalant, the intercalate representing a clay-chemical complex wherein the clay gallery (interlayer) spacing has increased, due to swelling resulting from surface modification by an intercalant, which often involves cation exchange processes. Under the proper conditions of temperature and shear, an intercalate is capable of exfoliating in a resin matrix, wherein the lamellar-structure of the clay is degraded, and the clay ultimately exfoliates into individual sheets. An intercalant is an organic or semi-organic chemical capable of entering the clay gallery and bonding to the surface. Exfoliation describes a dispersion of a surface treated nanoclay in a plastic matrix. In exfoliated form, nanoclay platelets have a flexible sheet-type structure, which is remarkable for its very small size, especially the thickness of the sheet is astoundingly small, measuring only about a nanometer. The length and breadth of the particles range from 1.5 µm down to a few tenths of a micrometer. These dimensions result in extremely high average aspect ratios (75-500). Moreover, the miniscule size and thickness mean that a single gram contains over a million individual particles. The nanoclays may be used in the form of nanocomposites, i.e. a combination of a surface treated nanoclay and a resin matrix.

Clays are commonly provided as particles having compositions based on hydrated aluminum silicates. The dimensions of the clay particles are typically in the range from 100 nanometers (nm) to 10 microns. Certain clay particles have structures containing multiple layers or stacks of clay platelets. Under suitable conditions, the stacks of clay platelets can be partially or completely separated to individual clay platelets. As used herein, the term "exfoliated clay" refers to a clay in the form of separated clay platelets having only one dimension in the range of nanometers and the other two dimensions in a larger size range, such as 100 nanometers and greater. As used herein, the term "to exfoliate" refers to the process of separating individual platelets from a clay particle or a stack of clay platelets, wherein the clay platelets have only one dimension in the range of nanometers and the other two dimensions in a large size range, such as 100 nanometers and greater. Typical size ranges for the exfoliated clays are platelets having one dimension, referred to as the thickness, in the range of from 1 nm to 20 nm, preferably in the range of from 1.5 nm to 15 nm, and more preferably in the range of from 2 nm to 12 nm. The other two dimensions of the platelet, which form the two faces of the platelet, are larger than the platelet thickness, and are typically in the range of from 50 nm to 20 microns, preferably in the range of from 75 nm to 15 microns, and more preferably in the range of from 100 nm to 10 microns. Surface areas for a platelet face are typically in the range of 2500 square nanometers to 400 square microns.

A clay suitable for use as nanostructured sorption material in aspects of the present invention typically has a layered structure. A suitable clay type has a cation exchange capacity of between 30 and 250 milliequivalents per 100 gram. If a clay with a cation exchange capacity higher than 250 milliequivalents per 100 gram is used, it will be difficult to finely disperse the clay at a molecular level due to the strong mutual interaction between the clay layers. If a clay with a cation exchange capacity lower than 30 milliequivalents per 100 gram is used, it will be difficult to modify the clay due to the fact that the interaction with the polymer will be weak. Preferably a clay having a cation exchange capacity of between 50 and 200 milliequivalents per 100 gram is used. Very suitable are clays based on layered silicates, such as layered phyllosilicatescomposed of magnesium and/or alumina silcate layers, each having a thickness of 7-12 Å. The clay may have a natural or synthetic origin. Preferably the clay has a large contact surface. Very suitable calys are clays from the synthetic small smectites and/or smectite-like clays such as montmorillonite, hectorite, fluorohectorite, beidellite, nontronite, bentonite, saponite, vermiculite, halloysite and/or stevensite.

The nanoclay is preferably an exfoliated nanoclay.

The nanoclay used in aspects of the invention may be a nanocomposite. There are in principle three possible structures when nanoclays are added to polymers: i) phase separated, wherein the nanofillers are intact and phase separated from the polymer, ii) intercalated, wherein the polymer has entered the clay gallery; and iii) exfoliated, wherein the clay platelets are dispersed in the polymer. The latter two are forms of nanocomposites. Nanocomposites are compositions containing a dispersed material that has one or more dimensions, such as length, width, or thickness, in the nanometer size range. Thus, polymer-clay nanocomposites typically are characterized as being one of several general types: an intercalated nanocomposite, an exfoliated nanocomposite, or combinations thereof. The term "intercalated nanoclay" as used herein, describes a nanoclay that is characterized by the regular insertion of a polymer in between the clay layers, wherein the individual clay platelets are not completely separated from the clay particle. The term "exfoliated nanoclay", as used herein, describes a nanocomposite wherein the clay is dispersed in a polymer matrix mostly as individual platelets having a single dimension, the thickness, in the nanometer size range. The exfoliated nanocomposite maximizes the polymer-clay interactions as the entire surface of the clay platelet is in contact with the polymer matrix. This modification often leads to the most dramatic changes in the mechanical and physical properties of the resultant polymer. In contrast, a conventional (phase separated) composite is a composite in which the clay acts as a conventional filler and is not dispersed on a nanometer size scale. In certain embodiments of the present invention, some portion of the clay in the polymer-clay nanocomposite optionally exists as structures larger than exfoliated or intercalated composites.

The (polymer) nanocomposites used in aspects of the present invention may suitably be based on clays, layered silicates and layered double hydroxides (hydrotalcites). The nanocomposites are typically obtained by the intercalation or penetration of the polymer (or a monomer subsequently polymerized) inside galleries of layered clay material and optionally the subsequent exfoliation, or dispersion, of the intercalate throughout the polymer matrix.

The term "nanostructured material" (also interchangeably referred to herein as "nanomaterial" or "nanostructured sorption material" ) is defined herein as a material having at least one dimension in the nanometer-size. A nanometer (nm) is 10⁻⁹ meter, therefore, nanometer-size range encompasses from about 1 to 999 nm. The nanostructured materials may be natural, modified, or synthetic in nature, or any combination thereof. Nanostructured materials particularly suitable for use in aspects of the invention include one or more of the following categories of nano-sized materials: nanoparticles or nanocrystalline materials, nanoporous materials, nanocomposites, nanoclays and nanofibers (nanotubes and nanowires), and any combination thereof. A nanostructured material might, for example, contain a single nanocrystalline material or it might contain two nanocomposites combined with a type of nanoparticle.

Nanocrystalline materials (the terms nanoparticles, nanocrystalline materials and nanopowders are used interchangeably herein) are nano-sized crystallites, preferably about 1 to 10 nm in dimension, and having an ultrahigh surface-to-volume ratio. The nanocrystalline materials may be synthesized *de novo* by precipitation or may be prepared by the size reduction of larger particles.

The use of nanocrystalline materials, for instance based on metal oxides and metal hydroxides for deodorizing an enclosed space is e.g. known from WO 2007/051145. Preferred nanocrystalline materials for use in connection with the present invention include the metal oxides and metal hydroxides of Mg, Sr, Ba, Ca, Ti, Zr, Fe, V, Cr, Co, Y, Mn, Ni, Cu, Al, Si, Zn, Ag, Au, Mo, Sb, Ce and mixtures thereof. The nanocrystalline materials may be optionally coated as disclosed in US 6,093,236, and US 5,759,939, may be halogenated as disclosed in US 6,653,519, US 6,087,294 and US 6,057,488 or in other ways modified as described in e.g. US 6,887,302 and US, all of which are incorporated by reference herein. The nanocrystalline materials preferably present crystallite sizes of less than about 25 nm, more preferably less than 20 nm, and most preferably less than 10 nm. The nanocrystalline particles preferably exhibit a Brunauer-Emmett-Teller (BET) multipoint surface area of at least about 100 m²/g, more preferably at least about 300 m²/g, and most preferably from about 700 m²/g and more. Exemplary nanocrystalline materials are available from NanoScale Materials, Inc., Manhattan, Kansas, under the name NanoActive®.

Nanopowder materials, which term refers in particular to nanostructured metal powders, preferably nanopowders of metal oxides or nanometal hydroxides, may for instance be produced by the reduction of metal salts, oxides or hydroxides to a powder consisting of ultrafine metallic or non-metallic particles in the nano-scale range (5-100 nm). Nanopowders of metal oxides or hydroxides can for instance be produced by subjecting a solution of a salt of the metal (generally a chloride) in a suitable solvent (e.g. water) to ultra sound waves (up to 0.6 W/cm3) in the presence of a base, such as e. g. an alkali hydroxide. Under such conditions, highly active radicals are rapidly created inside cavitation bubbles, that explode rapidly, leaving nuclei of nano-particles (for more details see e.g. WO 2003/012800). Examples of nanoparticle compounds which can be produced in this way include nanoparticles of metals, metal oxides and metal hydroxides. In principle, any metal may be used including but not limited to Mg, Sr, Ba, Ca, Ti, Zr, Fe, V, Cr, Co, Y, Mn, Ni, Cu, Al, Si, Zn, Ag, Au, Mo, Sb, Ce etc. and combinations thereof. Also non-metals can be included in the nanopowder material, and for instance hydroxyapatite (pentacalcium hydroxide triphosphate) may be produced in nanopowdered form. Alternatively nanopowder materials may be produced as inorganic nanoparticles using flame-spray synthesis technology (e.g. US 2006/162497).

Nanoporous materials are characterized by the molecular assembly of structures consisting of nanometer-sized cavities or pores. Nanoporous materials for use in the present invention may include nanoporous silica's, nanoporous alumina silicates (such as zeolites). The nanoporous materials may be based on natural materials or may be synthetically prepared.

Very suitable nanoporous materials for use in the present invention are metal organic frameworks (MOFs). Metal organic frameworks are hybrid materials where metal ions or small nano-clusters are linked into one-, two- or three-dimensional structures by multi-functional organic linkers as described, for example, in US 5,648,508 and US 6,893,564. Among the advantages of these materials are: (i) larger pore sizes can be realized than for the zeolites used presently (ii) the internal surface area is larger than for porous materials used presently (iii) pore size and/or channel structure can be tailored over a large range, (iv) the organic framework components forming the internal surface can be functionalized easily. In many cases it is possible to obtain open micro- and mesoporous structures having high porosities and specific surface areas of even above 5000 m2/g. Such open, porous, structures are very suitable for use as nanostructured adsorbents in the present invention. In addition it is possible to use conventional organic procedures to introduce a variety of functional groups in the organic part of the framework, thereby tailoring the affinity towards reactants and host molecules. The metal organic frameworks for use in the present invention may be based on Mg, Sr, Ba, Ca, Ti, Zr, Fe, V, Cr, Co, Y, Mn, Ni, Cu, Al, Si, Zn, Ag, Au, Mo, Sb, Ce or any other metal that provides good adsorption characteristics. The preferred metals in applications for dialysate fluid or blood (plasma) purification in nanostructured materials used in aspects of the present invention are preferably based on metals such as Fe, Ti and Mg. Other metals could be harmful to the body when inadvertently released form the matrix.

Other very suitable nanomaterials for use in aspects of the invention are "zeolite imidazolate frameworks" (ZIFs), composed of tetrahedral Si(Al)O₄ units covalently joined by bridging O atoms to produce a wide variety of frameworks. These frameworks may be functionalized such as for instance described in US 2007/0202038.

Typical nanostructured materials of the present invention may be based on aluminosilicates. Aluminosilicate nanostructured materials include, but are not limited to, layered polysilicates such as magadiite and kenyaite, polysilicates such as wollastonite, phyllosilicates such as the smectite group of clay minerals, tectosilicates such as zeolites, tetrasilicates such as kenyaite, and zeolites. Natural or synthetic phyllosilicates, for example, are sheet structures basically composed of silica tetrahedral layers and alumina octahedral layers. Phyllosilicates are one of the preferred types of nanostructured material, and a preferred type of phyllosilicate includes one or more smectite clays alone or in combination with other compatible structured nanomaterials. Additional examples of phyllosilicates useful in the present invention include, but are not limited to, montmorillonite, nontronite, beidellite, hectorite, saponite, sauconite, kaolinite, serpentine, illite, glauconite, sepiolite, vermiculite, or mixtures thereof. Though not restricted in particular, the total cation exchange capacity of the phyllosilicates can preferably be 10 to 300 milliequivalents, more preferably from 50 to 200 milliequivalents, per 100 grams of the phyllosilicate material. Phyllosilicate nanomaterials (i.e., nanoclays) are commercially available from Nanocor, Inc. of Arlington Heights, Ill. as NANOMER and from Southern Clay Products, Inc. of Gonzales, Tex. as CLOSITE.

Tectosilicates and tetrasilicates are another class of synthetic or natural aluminosilicates that are crystalline porous nanostructures having long-range crystalline order with pore sizes that may be varied from about 2 Å to 200 Å (Angstroms). The zeolite aluminosilicates may be divided into those with a fibrous habit and an underlying chain structure (i.e., natrolite); those with a platy habit and an underlying sheet structure (i.e., heulandite); and those with an equant habit and an underlying framework structure (i.e., chabazite). Zeolites may be synthesized by any technique known to those who are skilled in the art, such as that taught in U.S. Pat. No. 5,098,684, which is incorporated herein by express reference thereto.

The nanostructured material may be a carbonaceous nanomaterial. Carbonaceous nanomaterials suitable for use in aspects of the present invention include fullerenes, carbon nanoparticles, diamondoids, porous carbons, graphites, microporous hollow carbon fibers, single-walled nanotubes and multi-walled nanotubes. Fullerenes typically consist of 60 carbon atoms joined together to form a cage-like structure with 20 hexagonal and 12 pentagonal faces symmetrically arrayed. Preferred fullerene materials include C₆₀ and C₇₀, although other "higher fullerenes" such as C₇₆, C₇₈, C₈₄, C₉₂, and so forth, or a mixture of these materials, could conceivably be employed. Graphite is a crystalline form of carbon comprising atoms covalently or metallically bonded in flat layered planes with weaker van der Waals bonds between the planes.

Diamondoids are three-dimensional polycyclic organic compounds that may be substituted or unsubstituted. The term "lower diamondoids" refers to all isomers and stereoisomers of adamantane, diamantane, triamantane, or mixtures thereof. The term "higher diamondoids" refers to all isomers and stereoisomers of tetramantane, pentamantane, hexamantane, heptamantane, octamantane, nonamantane, decamantane, undecamantane, or mixtures thereof. The lower diamondoids may be synthesized or refined from a natural gas stream in a two stage separation process, e.g., as described in U.S. Pat. No. 4,952,748, the disclosure of which is incorporated herein by express reference thereto. The higher diamondoids may be isolated or purified from natural gas condensates or refinery streams through pyrolysis, distillation, pyrolysis/distillation, preparative gas chromatography, high performance liquid chromatography, crystallization, recrystallization, thermal diffusion, or fractional sublimation, as described in WO 02/058139.

Carbon nanotubes may be a single layer or multiple layers of the hexagonal lattice graphite, wrapped into a cylindrical tube, typically of about 1 nanometer diameter, but up to several microns long. This gives an aspect (or length-to-diameter) ratio that can be in excess of 10,000. The nanotubes may be further grown into partially ordered two-dimensional bundles, or "ropes" formed from individual nanotubes. Carbon nanotubes are typically prepared from the decomposition of carbon-containing gases over selected catalytic metal surfaces at temperatures ranging from about 500° C. to about 1,200° C., such as that described in U.S. Pat. No. 6,517,800, the disclosure of which is incorporated herein by express reference thereto.

The nanostructured materials of the present invention can include refined but unmodified nanomaterials, modified nanomaterials, synthetic nanomaterials, or mixtures thereof. The nanomaterials of the present invention may be dispersed directly into the polymer matrix, or may be dispersed with further intercalation or exfoliation processing, or some combination thereof. The intercalation or exfoliation processes may be conducted before, during, or after mixing the nanomaterials with the polymer matrix. However, it is not intended that these methods be limited to any specific process or procedure, and any suitable intercalation or exfoliation or combination thereof available to those of ordinary skill in the art may be used in accordance with the invention described herein.

"Intercalation" is defined as the insertion of mobile guest species (atoms, molecules, or ions) into a crystalline host lattice that contains an interconnected system of empty lattice sites of appropriate size. Intercalation is typically reversible, meaning that the structural integrity of the host lattice is formally conserved in the course of forward and reverse reactions.

An intercalated nanostructured material may be prepared, e.g., by the reaction of one or more swellable nanomaterials with a swelling agent of one or more organic molecules or cations. The process to prepare the intercalated nanomaterials may be conducted in a batch, semi-batch, or continuous manner. Numerous methods to modify nanomaterials with organic cations, such as those taught in US 4,810,734, which is incorporated herein by express reference thereto, are known, and any of these may be used in the practice of this invention.

Organic molecules suitable as swelling agents include cationic surfactants such as ammonium, phosphonium or sulfonium salts; amphoteric surface active agents; derivatives of aliphatic, aromatic or arylaliphatic amines, phosphines and sulfides; and organosilane compounds. Other suitable swelling agents include protonated amino acids and salts thereof containing 2-30 carbon atoms such as 12-aminododecanoic acid, epsilon-caprolactam and like materials. A preferred swelling agent includes ammonium to effect partial or complete cation exchange. The intercalation process results in the development of intercalates which are more organophilic and which can be more readily exfoliated (dispersed) during admixture with a polymer to form an ionomeric nanocomposite. These intercalates are typically on the order of 1 nanometer thick, but about 100 to 1,000 nanometers across. This high aspect ratio, and the resulting high surface area, provides high reinforcement efficiency at low loading levels.

Intercalation may also be accomplished by dispersing the nanostructured materials in a solution containing an oxidizing agent, e.g., a mixture of nitric and sulfuric acid, as described in US 3,404,061, the disclosure of which is incorporated herein by express reference thereto. The intercalation solution contains oxidizing and other intercalating agents known in the art. Examples include those containing oxidizing agents and oxidizing mixtures, such as solutions containing one or more of nitric acid, potassium chlorate, chromic acid, potassium permanganate, potassium chromate, potassium dichromate, perchloric acid, and the like, or mixtures of a strong organic acid, e.g., trifluoroacetic acid, and a strong oxidizing agent soluble in the organic acid. The intercalation solutions may further include electron donors (e.g., alkali metals, alkaline-earth metals, lanthanides, metal compounds containing hydrogen or polar molecules, and aromatic compounds) or electron acceptors (halogens, halides, oxyhalides, and acids) in an organic solvent. After intercalation, excess solution is removed, typically by driving off the solution by heat treating the mixture. In addition, the intercalation process may use an electrolytic intercalation solution in which the nanostructured material is subjected to electrolysis, dried, and then heated to temperatures up to 1,000 °C.

A sorbent material of the present invention, comprising a nanostructured sorption material captured in a porous polymer matrix, may be prepared by providing the nanostructured sorption material and the porous polymer matrix separately and combining the two such that the nanostructured sorption material is inserted in the porous polymer matrix. This may for instance occur by instilling the polymer matrix with a suspension of the nanostructured material.

Alternatively, in order to obtain a polymer matrix having a nanostructured material dispersed therein, one may prepare the matrix having the desired porosity, and synthesize the nonostructured material therein. Very suitable the polymer matrix may be imbibed with a solution of a metal salt which may then be precipitated the metal salt in the form of a metal hydroxide or metal oxide in the matrix.

### The polymer matrix

The invention relates to a sorbent material comprising a polymer for retaining the nanostructured sorption material. In other to provide for a matrix having a specific pore size, the polymer in preferred embodiments is a cross-linked polymer. In other preferred embodiments the polymer is a charged polymer.

Polymers used in aspects of the invention may be synthetic or natural polymers. Natural polymers (biopolymers) suitably comprise a cross-linked carbohydrate or protein, made of oligomeric and polymeric carbohydrates or proteins. The biopolymer is preferably a polysaccharide. Examples of polysaccharides include α-glucans having 1,3-, 1,4- and/or 1,6-linkages. Among these, the "starch family", including amylose, amylopectin and dextrins, is especially preferred, but pullulan, elsinan, reuteran and other α-glucans, are also suitable, although the proportion of 1,6-linkages is preferably below 70%, more preferably below 60%. Other suitable polysaccharides include β-1,4-glucans (cellulose), β-1,3-glucans, xyloglucans, gluco-mannans, galactans and galactomannans (guar and locust bean gum), other gums including heterogeneous gums like xanthan, ghatti, carrageenans, alginates, pectin, β-2,1- and β-2,6-fructans (inulin and levan), etc. A preferred cellulose is carboxymethylcellulose.

Carbohydrates which can thus be used are carbohydrates consisting only of C, H and O atoms such as, for instance, glucose, fructose, sucrose, maltose, arabinose, mannose, galactose, lactose and oligomers and polymers of these sugars, cellulose, dextrins such as maltodextrin, agarose, amylose, amylopectin and gums, e.g. guar. Preferably, oligomeric carbohydrates with a degree of polymerization (DP) from DP2 on or polymeric carbohydrates from DP50 on are used. These can be naturally occurring polymers such as starch (amylose, amylopectin), cellulose and gums or derivates hereof which can be formed by phosphorylation or oxidation. The starch may be a cationic or anionic modified starche. Examples of suitable (modified) starches that can be modified are corn-starch, potato-starch, pee-starch, rice-starch, tapioca starch, banana starch, and manioc starch. Other polymers can also be used (e.g. caprolactone). In certain embodiments, the biopolymer is preferably a cationic starch, most preferably an oxidized starch (for instance C6 oxidized with hypochlorite). The oxidation level may be freely chosen to suit the application of the sorbent material. Very suitably, the oxidation level is between 5 and 55 %, most preferably between 25 and 35%, still more preferably between 28% and 32%.

Most preferably the oxidized starch is crosslinked. A preferred crosslinking agent is di-epoxide. The crosslinking level may be freely chosen to suit the application of the sorbent material. Very suitably, the crosslinking level is between 0.1 and 25%, more preferably between 1 and 5%, and most preferably between 2.5 and 3.5%.

Proteins which can be used include albumin, ovalbumin, casein, myosin, actin, globulin, hemin, hemoglobin, myoglobin, gelatin and small peptides. In the case of proteins, proteins obtained from hydrolysates of vegetable or animal material can also be used. Particularly preferred protein polymers are gelatin or a derivative of gelatin.

Also suitable mixtures of carbohydrates (e.g. copolymers) or mixtures of proteins can be used.

In order to provide for a charged polymer, the carbohydrate polymer may for instance be modified by oxidation, substitution with cationic functional groups or with carboxymethyl groups, or by esterification with e.g. acetyl groups. Particularly preferred carbohydrate polymers are chosen from the group consisting of starch or a derivative of starch, cellulose or a derivative of cellulose, pectin or a derivative of pectin.

The formation of the matrix is accomplished through covalent cross linking of the polymers. The cross linker for cross-linking the polymers and providing the matrix structure is - in the case of carbohydrate polymers - preferably chosen from the group consisting of divinyl sulphone, epichlorohydrin, a di-epoxide such as glycerol diglycidyl ether or butanedioldiglycidyl ether, sodium trimetaphosphate and adipic acid or derivatives thereof. Typical crosslinks are ether- and/or ester-links, where for the ester-links phosphate-esters are preferable. Alternatively, the carbohydrate polymer may be cross-linked by means of a cross-linking enzyme chosen from the group consisting of peroxidases, laccases, polyphenol oxidases, transglutaminases, protein disulfide isomerases, sulfhydryl oxidases, lysyl oxidases and lipoxygenases. Methods how to use these cross-linkers or cross-linking enzymes are well known in the art.

A method for producing a polymer for use in aspects of the present invention is for instance described in EP 1 628 529 and may comprise:
a) providing a polymer (very suitably in an aqueous solution or suspension) and a cross-linker or cross-linking enzyme,
b) mixing the polymer and cross-linker or cross-linking enzyme to provide a polymer crosslinking reaction mixture;
c) activating the cross-linking by addition of base or acid to the reaction mixture;
d) allowing for cross-linking to occur and allowing gelation of the cross-linked polymer;
e) breaking the gel resulting from step d) into smaller particles; and
f) drying the polymer particles from step e) and optionally grinding these dried polymer particles into finer polymer particles.

It will be understood that the cross-linking level can be controlled by controlling the concentration of the cross-linker or the cross-linking reaction time. The level of cross-linking and the number of cross-links per polymer molecule will largely determine the pore size of the resulting polymer matrix.

The porous nature of the polymer matrix of the present invention is a key aspect. Firstly, as a result of the porous structure, fluids can enter the matrix and come into contact with (will be exposed to) the nanostructured sorption material immobilized therein. Due to this exposure, the substances of which the removal from the fluid is sought will adsorb to the nanostructured sorption material. Secondly, the porous structure of the polymer matrix will allow for the trapping of molecules of which the removal from the fluid is sought such as billirubin. Thus, the pore size should be selected such that the entry and subsequent absorption or adsorption into the matrix of molecules whose removal from the liquid is sought is possible. Thirdly, and in contrast, the porous structure of the polymer matrix should preferably prevent the entry into the matrix of large molecules of which the removal from the fluid is not sought, such as albumin. Thus, the pore size is preferably selected such that the entry into the matrix of molecules whose removal from the liquid is not sought is impossible. The pore size is thus selected to suit the specific purification application, and may for instance be based on size exclusion of compounds of which the removal from the liquid is not sought but which should be selectively retained therein.

Since a single albumin molecule is approximately an 80 angstrom diameter sphere, a suitable pore size for the polymer matrix in applications for blood (plasma) or dialysate purification would be less than 80 angstrom (less than 8 nm).

Drying of the polymer particles and optional further grinding thereof into fine dried polymer particles facilitates storage capabilities of the sorbent material of the present invention. Generally a polymer particle size of 100 µm to 3000 µm (based on the size of the dried particle) is suitable. Polymer particle sizes are preferably around 200 -1000 µm.

The nanostructured sorption material may be added to any of the steps a) - f) above to load the polymer particles with the nanostructured sorption material.

The advantages of cross-linked polymers lie in its intrinsic stability as a result of cross-links in the matrix. A further important advantage is that cross-linking provides a three-dimensional lattice of the cross-linked polymer, in which the nanostructured sorption material can be "filled in". Moreover, the choice of components, i.e. the choice of polymer(s) and cross-linker(s) influence the three-dimensional structure of the vehicle and thus would allow for the manufacture of specific matrices suited for retaining or excluding molecules of a certain size and/or certain charge.

The polymer matrix may be constructed from readily available and water soluble polymers such as polysaccharides and (hydrolysed) proteins and in doing so a flexible matrix may be formed and positive and/or negative charge through e.g. carboxylic acids and/or cationic groups will generate a custom made sorbent matrix. This cannot be accomplished using polysaccharides such as chitin and/or chitosan. Also the above mentioned polymers are much cheaper than the hitherto used chitin and chitosan. The possession of a charge is an important feature of a polymer for the present invention. It will greatly facilitate the formation of a complex between the nanostructured sorption material (which is often a charged molecule) and the polymer lattice. The charge can be provided by the polymer itself, but - if the polymer does not have a positive or negative charge - the charge can be introduced as a result of modification of the polymer or by the cross-linker used for cross-linking the polymer.

Modification of the polymers can be accomplished by oxidation, substitution with cationic functional groups or carboxymethyl groups and/or esterifying with e.g. acetyl groups. Although in the latter case no charge is added, it is used to make the polymer more hydrophobic to allow complexing of the polymer with nanostructured sorption materials that have little or no charge.

Generally the polymers will be modified before cross-linking and gelation. It is possible to modify the polymer after cross-linking and gelation only if cross-linking is performed by ether-formation. The person skilled in the art will know how to modify the polymers specified in the invention to provide them with the mentioned groups.

The charge of the cross-linked polymer can be negative or positive depending on the type of polymer, the type of modification and the type of cross-linking.

Advantageously, the polymers are of considerable size, i.e. 30 kD or more. This allows for the ready formation of a gel upon cross-linking and it allows for the formation of a lattice, which is capable of taking up the nanostructured sorption material.

In this way sorbent materials are formed that are stable and can be used in the various applications according to the invention. The sorbent materials will not gelate again when solved, even not when heated or boiled, and they do not spontaneous fall apart which would cause release of any nanostructured sorption material.

The size of the sorbent material particles depends on the breaking and grinding process. Breaking is preferably done by pressing the gel through a sieve of a desired mesh size. If necessary, finer particles can be formed by additional grinding the sieved particles, preferably after drying. The size of the sorbent material particles preferably can range from 0.5 µm to 1000 µm and the optimal size will depend on the specific application for which they are used. It is generally thought that small sorbent material particles are preferable for applications where contact time with the fluid from which purification is sought is short.

It is thought that loading of the nanostructured sorption materials is possible because complexes are formed due to electrostatic interactions between the charged groups of the cross-linked polymer and the charged groups on the nanostructured sorption materials. In the case that neutral components and/or polymers are used complex formation will probably be caused by hydrostatic interactions between hydrophobic groups.

In many cases, the nanostructured material will form a nanocomposite with the polymer matrix.

The selectivity of the sorbent material of the present invention can further be enhanced by loading the material with specific molecule catchers or receptors (general: binding partners). For selective binding of proteins or degenerated proteins, antibodies are very suitable, e.g. from the immunoglobulin superfamily (IgSF), prosthetic groups e.g. from lipid and vitamin derivatives or metal ion such as gold, iron, zinc, magnesium, calcium that covalently bond to proteins, as well as carboxyl groups that can bind to proteins by forming peptide bonds.

Alternatively, the nanocomposites may comprise amorphous or crystalline Hydrated Fe Oxide (HFO). Such materials exhibit strong sorption affinity towards toxic compounds such as arsenates, arsenites, chromates, molybdates, selenites, and vanadates, but also phosphates. A very suitable material for inclusion in the sorption material of the present invention is for instance disclosed in US2005/156136. This publication is expressly referred to and incorporated herein in its entirety by reference. US2005/156136 discloses the formation of an adsorbent comprising HFO particles supported in an polymeric anion exchange material. This material is capable of selective removal of certain toxic compounds as mentioned above from a fluid stream brought into contact with the adsorbent. The particles are formed by passing a solution containing an oxidizing anion such as a permanganate, persulfate or hypochlorite, through a bed of polymeric anion exchange resin (e.g. A-500P having quaternary ammonium functional groups in chloride form). Thereafter a solution of a ferrous salt, such as ferrous sulfate, is passed through the bed, thereby simultaneously desorbing the oxidizing anion and oxidizing the ferrous ion to a ferric ion. This causes precipitation, and uniform dispersion, of a solid, hydrated ferric oxide, within the polymeric anion exchange resin. Anionic ligands such as arsenates, chromates, oxalates, phosphates, phthalates, etc., can permeate in and out of the gel phase and are not subjected to the Donnan exclusion effect. Although the polymeric matrix used in the present invention may comprise HFO particles dispersed therein (preferably hydrated Fe(III) oxides), the matrix may also comprise particles of an anion exchange matrix comprising HFO particles disperses therein (a gel matrix comprising particles in particles). The advantage of such an embodiment is that a negative charge of a gel matrix supporting nanostructured sorption materials according to the present invention that would normally repel negatively charged compounds such as arsenates and phosphates, can still support the effective removal of such compounds from a fluid feed if the HFO particles are dispersed in an anion exchange matrix.

### Applications of the sorbent material of the invention

The sorbent material of the present invention may be used in a large number of fluid purification applications.

For instance the sorbent material may be used to remove toxic chemicals or hormones from water.

The sorbent material may be used to remove toxic chemicals such as snake venom, arsenic or other substances from the blood of a patient.

The sorbent material may be provided in the form of particles and may be used as a dialysate additive, wherein the sorbent material as defined herein as an active ingredient for sorption of toxic substances from the dialysate fluid. A dialysate additive of the present invention is added to the dialysis fluid of a hemodialysis or peritoneal dialysis system enabling removal of toxins from the dialysis fluid. The sorbent material continuously purifies the dialysate fluid, keeping the toxin concentrations in the dialysis fluid low. This results in an improvement of the dialysis efficiency, typically with 100%, and reduces the consumption of dialysis fluid needed dramatically, ideally with 300%.

Alternatively, the sorbent material may be used as (part of) a sorption filter, and may for instance be enveloped by a hemofilter to provide for a filter pad for use in a artificial kidney system or hemofiltration unit (see Figs 1 and 7).

An additional and optimal function of the sorbent material in dialysis and blood purification applications is to release ingredients for supplementing of the blood such as calcium, vitamin A, anti-coagulants, minerals, antimicrobial agents, specific medicaments etc. This option will be beneficial to the operation of existing hemodialysis and peritoneal dialysis systems and will reduce the chance on occurring infections in the peritoneal dialysis system.

A sorbent material of the present invention may, in any embodiment, further comprise means for selective sorption of middle molecules, vitamins and minerals such as calcium, sodium and potassium. In order to facilitate this selective sorption, the polymer matrix or nanostructured sorption material may thereto be loaded with a certain amount of minerals, vitamins and can only absorb a designated (selective) amount.

Optionally, the sorbent material may comprise ion exchange systems in addition to the nanostructured sorption material.

In another aspect, the present invention provides a method for removing toxic substances from blood, blood pasma, hemodialysates or peritoneal dialysates, comprising using a sorbent material according to the present invention as a dialysate additive or as part of a sorption filter.

The sorbent material of the present invention may be formulated in any form, such as for instance in the form of a sol, a gel, a suspension, a powder, a microcapsule, a pellet, or a tablet. The formulation may comprise excipients, such as buffers, polymers, vitamins, carbohydrates, amino acids, minerals, salts, albumin, surfactants, anticoagulants, antimicrobials, medicaments or bulking agents.

The methods and uses of the present invention are *i*.*a*. distinguished from the prior art methods and uses in that use is made of nanomaterials to allow for the use of small amounts, typically from 5-30 grams/liter, as a result of which dispersion of the sorbing material in the dialysis fluid is possible. This will also allow multiple sorbent stages for removing multiple components (i.e. multiple substances from the blood), both from cationic as well as anionic nature.

In a preferred peritoneal system, the peritoneal dialysate fluid comprises a fluid based on advanced osmotic pressure generating substances such as albumins or polyols (e.g. sorbitol, xylitol, mannitol, glycosyl). The albumin or polyol based system has superior osmotic pressure diffeerence properties but it is rarely used because of higher costs compared to the standard glucose system. The present invention allows a significant reduction in dialysis fluid, enabling the economic use of advances osmotic substances. This will result in better clearance rates.

The sorbent material of the present invention can be used in dialysis fluids for peritoneal and hemodialysis systems, both in central clinics as well as in home-based systems. The material may also be packaged into a filter for use in a hemodialysis machine.

The sorbent material has a temporary use until it reaches its maximum capacity. The content of the sorbent material can be customized to the individual patient needs.

The invention will now be illustrated by way of the following nonlimiting examples.

### EXAMPLES

### Example 1

**Adsorbent:** nanoclay EXM1064 (Sud-Chemie AG, Munich, Germany), composition (Na_{0,2},Ca_{0,1})(Al,Mg)₂(Si₄O₁₀)(OH)₂

**Test fluid**: human urine

**Procedure:** 20 ml of urine, adjusted to a pH of about 7, was mixed with 0.5 gram of nanoclay in a glass container at standard conditions (20°C). Exposure time was 1 hr. Toxin concentrations in urine were measured before and after exposure. The table shows the initial concentration of the toxins in urine (in mmol/1) and the amount adsorbed by the adsorbent (in g/100 g of the adsorbent). The two tables provide data for different urine samples with two different toxin concentrations. It is shown that the amount of toxin adsorbed is dependent on the toxin concentration. The tables clearly indicate the amount of toxin that can be adsorbed by an amount of 100 grams of adsorbent

**Results:** the sorption results are expressed in sorped weight per component in grams per 100 gram sorption material (g/100g). The higher the amount of toxin in the urine, the higher the amount adsorbed in a time span of 1 hour.

**Table 1 (Control values)**

| | Concentration in urine (mmol/1) | Sorption after 1 hr (g/100g) |
|---|---|---|
| Potassium | 44 | 2.0 |
| Phosphate | 8 | 0.1 |
| Urea | 154 | 2.0 |
| Uric acid | 1.34 | 0.5 |
| Creatinine | 5.1 | 0.7 |
| Beta2microglobuline | 40 (µg/l) | >0.00014 |

**Table 2**

| | Concentration in urine (mmol/l) | Sorption after 1 hr (g/100g) |
|---|---|---|
| Potassium | 20 | 1.2 |
| Phosphate | 23 | 0.3 |
| Urea | 175 | 2.2 |
| Uric acid | 1.2 | 0.4 |
| Creatinine | 7.2 | 1.3 |
| Beta2microglobuline | 76 (µg/l) | 0.0003 |

### Example 2

**Absorbent:** hydrotalcite, composition Mg₂Fe(OH)₆.OH

**Preparation:** A solution of 25.63g of Mg(NOg)₂.6H₂O (Fluka, Sigma-Aldrich Chemie GmbH, Buchs, Switzerland ) and 20.40g of Fe(NO3)3.9H2O (Acros Organics, Geel, Belgium) in 70 ml demiwater was mixed at room temperature with 150ml of 2.5M NaOH (Aldrich) under vigorous stirring with an Ultra Turrax at 15000-20000 rpm. After 5 minutes of homogenizing, the mixture was subjected to a hydrothermal treatment for 7 hrs at 100°C. After filtration and washing, the filtrated material was dried in a stove at 80°C.

**Test fluid:** human urine

**Procedure:** 20 ml of urine, adjusted to a pH of about 7, was mixed with 0.5 gram of the hydrotalcite in a glass container at normal conditions (20 C). Exposure time is 1 hr. Toxin concentrations in urine were measured before and after exposure to the adsorbent.

**Results:** the sorption results are expressed in sorped weight per component in grams per 100 gram sorption material (g/100g).

**Table 3**

| | Concentration in urine (mmol/l) | Sorption after 1 hr (g/100g) |
|---|---|---|
| Potassium | 20 | 0.3 |
| Phosphate | 23 | >8.0 |
| Urea | 175 | 3.4 |
| Uric acid | 1.2 | 0.6 |
| Creatinine | 7.2 | 0 |
| Beta2microglobuline | 76 (µg/l) | 0.0003 |

### Example 3

**Absorbent:** Modified starch, oxystarch containing 15 wt% hydrotalcite, composition Mg.OH

**Preparation:** A solution of 3.16g of MgCl₂.6H₂O (JT.Baker, Mallinckrodt Baker, Inc., Phillipsburg, U.S.A.) in 10.27 ml of boiled demiwater is mixed at room temperature with a solution of 4.0g of Oxystarch (see Example 5) in 163 ml of boiled demiwater. After 10 minutes, 6 ml of a 33wt% NH₄OH solution (JT.Baker) was added. This mixture was stirred overnight. After filtration and washing, the filtrated material was dried in a stove at 80°C.

**Test fluid:** human urine

**Procedure:** 20 ml of urine, adjusted to a pH of about 7, was mixed with 0.5 gram of the starch/hydrotalcite in a glass container at normal conditions (20 C). Exposure time is 1 hr. Toxin concentrations in urine were measured before and after exposure to the adsorbent.

**Results:** the sorption results are expressed in sorped weight per component in grams per 100 gram sorption material (g/100g).

**Table 4**

| | Concentration in urine (mmol/l) | Sorption after 1 hr (g/100g) |
|---|---|---|
| Potassium | 20 | 0,6 |
| Phosphate | 23 | 7,4 |
| Urea | 175 | 3,4 |
| Uric acid | 1,2 | 0 |
| Creatinine | 7,2 | 0 |
| Beta2microglobuline | 76 (µg/l) | 0 |

### Example 4

**Absorbent:** Modified carboymethyl cellulose (CMC) containing 10 wt% hydrotalcite, composition Mg₂Fe(OH)₆.OH

**Preparation:** A solution of 0.56g FeCl₃ (Aldrich) and 1.10g of MgCl₂.6H₂O (JT.Baker) in 23.56 ml of boiled demiwater was mixed under a nitrogen atmosphere at 70°C with a solution of 3.03g of carboxymethyl cellulose (CMC) (AKUCELL, Akzo Nobel Nederland bv, Arnhem, The Netherlands) in 200 ml of boiled demiwater. After 5 minutes, a 1M NaOH solution was added until the pH reached 9.5. This pH was kept constant for 2 hrs (additional titration of 1M NaOH) and then stirred overnight at 70°C. After filtration and washing, the filtrated material was freeze dried.

**Test fluid:** human urine

**Procedure:** 20 ml of urine, adjusted to a pH 7, was mixed with 0.5 gram of the CMC/hydrotalcite in a glass container at normal conditions (20 C). Exposure time was 1 hr. Toxin concentrations in urine were measured before and after exposure to the adsorbent.

**Results:** the sorption results are expressed in sorped weight per component in grams per 100 gram sorption material (g/100g).

**Table 5**

| | Concentration in urine (mmol/l) | Sorption after 1 hr (g/100g) |
|---|---|---|
| Potassium | 10 | 0,3 |
| Phosphate | 12 | 3,7 |
| Urea | 88 | 1,2 |
| Uric acid | 0,6 | 0 |
| Creatinine | 3,6 | 0 |
| Beta2microglobuline | 38 (µg/l) | 0,001 |

### Example 5

**Absorbent:** Oxystarch, a cationic starch, C6 oxidized with hypochlorite (oxidation level estimated to 30%) and crosslinked with di-epoxide (estimated crosslinking 3%)

**Test fluid:** human urine

**Procedure:** 20 ml of urine, adjusted to a pH 7, was mixed with 0.5 gram of the sorbent in a glass container at normal conditions (20 C). Exposure time was 5 hr. Toxin concentrations in urine were measured before and after exposure to the adsorbent.

**Results:** the sorption results are expressed in sorped weight per component in grams per 100 gram sorption material (g/100g).

**Table 6**

| | Concentration in urine (mmol/l) | Sorption after 5 hr (g/100g) |
|---|---|---|
| Potassium | 20 | 0,2 |
| Phosphate | 23 | 0,2 |
| Urea | 175 | 2,9 |
| Uric acid | 1,2 | 0 |
| Creatinine | 7,2 | 0 |
| Beta2microglobuline | 76 (µg/l) | 0,00001 |

## Claims

1. A sorbent material for removing substances from an aqueous liquid comprising a nanostructured sorption material captured in a porous polymer matrix, wherein the pores in said matrix are of a size that allows the entry into said matrix of substances sought to be removed from said liquid while preventing the escape of said nanostructured sorption material or preventing the entry into said matrix of substances not sought to be removed from said liquid.

2. Sorbent material according to claim 1, wherein the nanostructured sorption material is selected from the group consisting of nanoparticles or nanocrystalline materials, nanoporous materials, nanocomposites, nanoclays and nanofibers, and any combination thereof.

3. Sorbent material according to claim 2, wherein said nanocrystalline material is a nano hydrotalcite.

4. Sorbent material according to claim 2, wherein said nanoclay is an exfoliated nanoclay

5. Sorbent material according to claim 2, wherein said nanoporous material is selected from zeolites, mesoporous systems, metal organic frameworks.

6. Sorbent material according to any one of the preceding claims wherein said porous polymer matrix is based on a cross-linked polymer and/ or a charged polymer.

7. Sorbent material according to claim 6, wherein said polymer is a biopolymer selected from carbohydrates and proteins.

8. Sorbent material according to claim 7, wherein said carbohydrate is an oxidized crosslinked starch.

9. Sorbent material according to any one of the preceding claims, wherein the aqueous liquid is dialysate fluid, blood or bloodplasma.

10. Sorbent material according to any one of the preceding claims, wherein said substances sought to be removed from said liquid are toxic substances.

11. Sorbent material according to claim 10, wherein said toxic substances are selected from potassium, phosphate, urea, creatinine, beta2-microglobulin (β2M), and albumin bound toxins.

12. Sorbent material according to any one of the preceding claims, wherein said sorbent material further comprises means for supplementing said dialysate fluid and/or said (purified) blood plasma with at least one substance selected from the group consisting of vitamins, minerals, anticoagulants, anti microbial agents and medicaments.

13. Sorbent material according to any one of claims 1-12, for removing toxins, small and middle-sized molecules from a dialysate or a patient's blood or blood plasma.

14. Sorbent material according to any one of claims 1-12, for use as an additive to dialysate fluids.

15. Sorbent material according to any one of claims 1-12, for use in a hemodialysis system or peritoneal dialysis system.

16. Filter pad comprising a sorbent material according to any one of claims 1-12, and further optionally comprising a hemofilter.

17. Filter pad according to claim 16, wherein the sorbent material is provided in the form of dried granules having a mean size over the range 250 microns to 1500 microns in dried form.

18. A method for removing substances from an aqueous liquid comprising the step of exposing said liquid to a sorbent material according to any one of claims 1-12 or filtering said liquid through a filter pad according to claim 16 or 17.
